# EUROPÄISCHE PATENTSCHRIFT

(11) **EP 3 202 325 B1**
(45) Veröffentlichungstag und Bekanntmachung des Hinweises auf die Patenterteilung: **10.04.2019**
(21) Anmeldenummer: 17168761.9
(22) Anmeldetag: 28.04.2017
(51) Int. Cl.: A61B 6/00

(54) **TRAGEVORRICHTUNG**
CARRYING DEVICE
DISPOSITIF DE SUPPORT

(43) Veröffentlichungstag der Anmeldung: 09.08.2017
(73) Patentinhaber: Siemens Healthcare GmbH, 91052 Erlangen (DE)
(72) Erfinder: Krämer, Alexander, 92699 Irchenrieth (DE); Meyer, Michael, 91353 Hausen (DE)

(56) Entgegenhaltungen:
- DE-A1-102010 020 603
- DE-A1-102012 217 072
- US-A1- 2014 177 799

## Beschreibung

Die Erfindung betrifft ein Röntgensystem.

In der Medizintechnik sind oft Tragevorrichtungen im Einsatz, die mit Haltevorrichtungen verbunden sind und entweder an einer Decke oder dem Boden aufgehängt sind. Ein Röntgensystem weist typischerweise zumindest eine Tragevorrichtung und eine Haltevorrichtung für eine Röntgeneinheit auf. Die Röntgeneinheit weist typischerweise einen Röntgenstrahler und einen Röntgendetektor auf.

Bei einigen Anwendungen, beispielsweise in der interventionellen Angiographie oder Koronarangiographie, ist mitunter die Tragevorrichtung mittels einer motorischen Fernsteuerung variabel einstellbar. Eine derart ausgebildete Tragevorrichtung kann die Position der daran angeordneten Haltevorrichtung kontinuierlich oder diskret in mindestens einer Raumrichtung oder um zumindest eine Raumachse herum verändern oder lösbar feststellen. Dies dient insbesondere der Positionierung und Ausrichtung der Haltevorrichtung. Üblicherweise kann ebenso die Röntgeneinheit flexibel um den auf einer freitragenden Tischplatte, insbesondere einer Patientenliege, angeordneten Patienten herum eingestellt werden. Vorzugweise kann daher mittels der Röntgeneinheit eine optimale Behandlung eines Patienten durchgeführt werden.

Es sind Tragevorrichtungen bekannt, die entweder an der Decke oder dem Boden aufgehängt sind und mit einer Haltevorrichtung verbunden sind, wobei die Haltevorrichtung einen C-Bogen mit einer an dessen Enden angeordneten Röntgeneinheit aufweist. Die Haltevorrichtung weist oft weitere Elemente auf, die der Ausrichtung der Röntgeneinheit dienen. Die Aufhängevorrichtung kann am Boden oder der Decke eines Untersuchungsraums entweder fest oder insbesondere auf Schienen gelagert verfahrbar eingerichtet sein.

In DE 10 2015 202 082 A1 ist ein deckengebundenes medizinisches Röntgengerät offenbart, wobei ein Röntgenstrahler und ein Röntgendetektor um eine Orbitalachse bewegbar ist mittels eines verfahrbaren C-Bogens und eines daran angeordneten G-Bogens.

Aus DE 10 2014 202 013 A1 ist eine Röntgenvorrichtung bekannt, die einen C-Bogen mit einem daran drehbar gelagerten Strahlungsdetektor umfasst, der über ein motorisches Antriebsmittel drehbar ist.

DE 10 2012 208 850 A1 beschreibt eine Röntgenvorrichtung zur Aufnahme von Strahlungsbildern, umfassend eine Strahlungsquelle und einen Strahlungsempfänger, die an einem gemeinsamen C-Bogen angeordnet sind, der an einer deckenseitig montierten Tragkonstruktion angeordnet ist, wobei der C-Bogen um ein Isozentrum drehbar an der Tragkonstruktion angeordnet ist, sowie eine im wesentlichen rechteckförmige Patientenliege und eine Steuerungseinrichtung, wobei die deckenseitig sowohl parallel zur Längsrichtung der Patientenliege als auch senkrecht zur Längsrichtung der Patientenliege bewegbare Tragkonstruktion zumindest senkrecht zur Längsachse auch während der mit rotierendem C-Bogen erfolgenden Bildaufnahme bewegbar ist.

Die DE 10 2012 217 072 A1 offenbart eine Vorrichtung zum Positionieren von Geräteeinheiten, insbesondere zum Positionieren von mobilen C-Bögen.

Die US 2014 0177799 A1 beschreibt ein Röntgenbildgebungssystem, welches einen C-Arm, an dessen Ende ein Röntgenstrahler und ein Röntgendetektor angeordnet sind, aufweist, wobei der C-Arm um eine Rotationsachse rotierbar ist, welche auf einer Aufhängevorrichtung des Röntgenbildgebungssystems senkrecht steht.
Aus der DE 10 2010 020 603 A1 ist eine Bildaufnahmevorrichtung bekannt, umfassend eine ringförmige Gantry mit einer in ihr rotierenden Rotoranordnung mit einer Strahlungsquelle, sowie mindestens einem Srahlungsdetektor, wobei die wenigstens ein aus der Ringform zum seitlichen Öffnen der Gantry herausnehmbares Gantrysegment aufweisende Gantry an einer Tragstruktur im Raum bewegbar angeordnet ist, wobei die Tragstruktur ein boden-, wand- oder deckenseitig montierter, wenigstens vier, vorzugsweise sechs Bewegungsfreiheitsgrade aufweisender Knickarmroboter ist, wobei in der Gantry wenigstens zwei um einen Winkel versetzt an der Rotoranordnung angeordnete Strahlungsquellen vorgesehen sind, denen jeweils mindestens ein Strahlungsdetektor zugeordnet ist.

Der Erfindung liegt die Aufgabe zugrunde ein Röntgensystem anzugeben, das eine flexible Bewegung der Haltevorrichtung ermöglicht.

Die Aufgabe wird durch die Merkmale des unabhängigen Anspruchs gelöst. Vorteilhafte Weiterbildungen sind in den abhängigen Ansprüchen angegeben.

Das erfindungsgemäße Röntgensystem weist
- eine Tragevorrichtung, welche
   -- eine Aufhängevorrichtung,
   -- ein an der Aufhängevorrichtung angeordnetes erstes Trägerelement,
   -- ein zweites Trägerelement,
   -- ein erstes Verbindungselement, wobei das zweite Trägerelement mittels des ersten Verbindungselements mit dem ersten Trägerelement verbunden ist,
   -- ein drittes Trägerelement und
   -- ein zweites Verbindungselement aufweist, wobei das dritte Trägerelement mittels des zweiten Verbindungselements mit dem zweiten Trägerelement verbunden ist, und
- eine mit dem dritten Trägerelement verbundene Haltevorrichtung auf, die einen C-Bogen, an dessen Enden ein Röntgenstrahler und ein Röntgendetektor angeordnet sind, und eine Verbindungsvorrichtung aufweist,
- wobei das erste Verbindungselement und/oder das zweite Verbindungselement ein Drehgelenk aufweist, der Abstand zwischen der Aufhängevorrichtung und dem ersten Verbindungselement mittels des ersten Trägerelements veränderbar ist und der Abstand zwischen dem ersten Verbindungselement und dem zweiten Verbindungselement mittels des zweiten Trägerelements veränderbar ist,
- wobei der Röntgenstrahler und der Röntgendetektor entlang einer von der Form des C-Bogens vorgegebenen ersten Bahn, die Teil eines Drehkreises ist und deren Bogenlänge einem ersten Winkel entspricht, mittels einer Bewegung des C-Bogens relativ zur Verbindungsvorrichtung verfahrbar angeordnet sind,
dadurch gekennzeichnet,
dass der Röntgenstrahler und der Röntgendetektor mittels des ersten Trägerelements, des Drehgelenks, des zweiten Trägerelements und der Bewegung des C-Bogens relativ zur Verbindungsvorrichtung entlang einer zweiten Bahn, die Teil des Drehkreises ist und deren Bogenlänge einem zweiten Winkel entspricht, verfahrbar angeordnet sind, wobei der zweite Winkel größer ist als der erste Winkel.

Die Tragevorrichtung kann derart eine multidimensionale Kinematik erlauben und daher das Verstellen der Tragevorrichtung im Raum, insbesondere eine flexible Positionierung des dritten Trägerelements, ermöglichen. Die Erfindung bietet daher insbesondere mehrere Vorteile:
1) Bei einer geeigneten Ausführung der Tragevorrichtung kann das dritte Trägerelement mittels der Tragevorrichtung insbesondere höhenverstellbar sein. Wenn eine Haltevorrichtung mit der Tragevorrichtung verbunden ist, kann vorzugweise mittels der Tragevorrichtung die Haltevorrichtung, insbesondere die Röntgeneinheit höhenverstellbar sein. Beispielsweise kann mittels der Tragevorrichtung ein vertikaler Isozentrumshub durchgeführt werden, wobei insbesondere das Isozentrum der Röntgeneinheit entlang einer Achse, welche insbesondere senkrecht auf dem Boden oder der Decke steht, auf einer geraden Linie verstellbar und/oder lösbar feststellbar ist.
2) Die Tragevorrichtung kann einen zusätzlichen Freiheitsgrad entlang des ersten Trägerelements aufweisen. Üblicherweise kann eine Tragevorrichtung eine Bewegung insbesondere der verbundenen Haltevorrichtung entlang des ersten Trägerelements nur dann ausführen, wenn die Aufhängevorrichtung auf Schienen gelagert ist. Bauartbedingt ist diese Bewegung der Aufhängevorrichtung entlang von Schienen oft nur in zwei Raumrichtungen möglich, welche bevorzugt senkrecht aufeinander stehen, wobei eine Raumrichtung beispielsweise parallel zu einer Patientenliege ist und eine andere Raumrichtung dazu senkrecht steht. Durch eine geeignete Anordnung des ersten Trägerelements an der Aufhängevorrichtung und eine entsprechende Ausführung der Tragevorrichtung erfolgt beispielsweise eine Verschiebung der Haltevorrichtung, insbesondere der Röntgeneinheit in eine Richtung, die parallel zu einer Decke oder zu einem Boden sein kann. Vorzugweise kann die Verschiebung der Haltevorrichtung in horizontaler Richtung erfolgen. Typischerweise kann die Verschiebung der Haltevorrichtung in eine zu der Decke oder zu dem Boden parallele Richtung ohne Bewegung der Aufhängevorrichtung relativ zu der Decke oder zu dem Boden erfolgen. Damit können beispielsweise außermittige Regionen des Patienten einfacher durchstrahlt werden.
3) Die Tragevorrichtung kann derart vorzugweise ausgebildet sein, dass das dritte Trägerelement insbesondere entlang einer Kreisbahn verschiebbar ist. Vorzugweise ist ein Ende des dritten Trägerelements, an welchem das dritte Trägerelement mit der Haltevorrichtung verbunden ist, entlang der Kreisbahn verschiebbar. Dies ist typischerweise möglich durch eine Kombination des Verstellens des Drehgelenks, dem Verändern des

Abstands zwischen der Aufhängevorrichtung und dem ersten Verbindungselement mittels des ersten Trägerelements und des Abstands zwischen dem ersten Verbindungselement und dem zweiten Verbindungselement mittels des zweiten Trägerelements.

Die Aufhängevorrichtung ist vorzugweise zur Anordnung, insbesondere zur Befestigung der Tragevorrichtung an einer Decke oder an einem Boden, insbesondere in einem Untersuchungsraum, ausgebildet. Typischerweise ist eine Befestigung der Aufhängevorrichtung an der Decke oder dem Boden lösbar. Die Tragevorrichtung ist üblicherweise derart ausgebildet, dass beispielsweise das erste Trägerelement mittels der Aufhängevorrichtung mit der Decke des Untersuchungsraums verbunden ist. Die Aufhängevorrichtung kann beispielsweise einen Deckenwagen aufweisen und derart ausgebildet sein, dass sie auf Schienen einer ersten Schienenebene gelagert ist. Dies kann insbesondere eine Verschiebung der Aufhängevorrichtung entlang der Schienen der ersten Schienenebene und Feststellung der Aufhängevorrichtung an den Schienen der ersten Schienenebene ermöglichen. Weiterhin ist es auch denkbar, dass die Aufhängevorrichtung derart auf einer zweiten Schienenebene gelagert ist, dass eine Verschiebung der Aufhängevorrichtung entlang der Schienen der zweiten Schienenebene und Feststellung der Aufhängevorrichtung an den Schienen der zweiten Schienenebene alternativ oder zusätzlich zum Verschieben auf der ersten Schienenebene erfolgen kann. Beispielsweise kann die Aufhängevorrichtung, insbesondere der Deckenwagen auf den Schienen der ersten Schienenebene gelagert sein, welche wiederum auf den Schienen der zweiten Schienenebene angeordnet sind. Der Deckenwagen kann geeignete Mittel aufweisen, um die Aufhängevorrichtung auf der ersten Schienenebene oder der zweiten Schienenebene zu verfahren. Die erste Schienenebene oder die zweite Schienenebene können auch Teil der Aufhängevorrichtung sein. Vor allem können die erste Schienenebene und/oder die zweite Schienenebene derart ausgebildet sein, dass die erste Schienenebene relativ zur zweiten Schienenebene insbesondere verfahrbar sein kann. Vorzugweise sind die Schienen der Schienenebenen gerade ausgebildet. Üblicherweise sind die Schienenebenen parallel zum Boden oder zur Decke ausgerichtet. Insbesondere können die sich in Längsrichtung der Schienenebenen erstreckenden Längsachsen aufeinander senkrecht stehen. Daraus folgt, dass in diesem Fall die Aufhängevorrichtung in zwei Raumrichtungen verfahrbar sein kann.

Das erste Trägerelement ist an der Aufhängevorrichtung angeordnet. Diese Anordnung kann gemäß einer Befestigung ausgebildet sein. Typischerweise ist das erste Trägerelement derart an der Aufhängevorrichtung angeordnet, dass das erste Trägerelement relativ zur Aufhängevorrichtung verfahrbar sein kann. Alternativ oder zusätzlich kann diese Befestigung starr sein. Eine Ausführungsform sieht vor, dass die Aufhängevorrichtung Mittel zur derartigen Anordnung des ersten Trägerelements umfasst, damit das erste Trägerelement um eine Systemachse der Tragevorrichtung, welche bei einer Aufhängung der Tragevorrichtung an der Decke oder dem Boden senkrecht auf der Decke oder dem Boden steht, drehbar gelagert ist. Üblicherweise verläuft die Systemachse durch die Aufhängevorrichtung, insbesondere durch einen Drehpunkt der Aufhängevorrichtung, um welchen das erste Trägerelement drehbar gelagert ist. Das erste Trägerelement kann daher drehbar um die Systemachse gelagert sein, wobei zumindest ein Ende des ersten Trägerelements auf einen Drehkreis verfahren werden kann, deren Drehkreis-Ebene planparallel zur Decke ist. Die vorteilhafte Höhenverstellbarkeit des dritten Trägerelements, die die Tragevorrichtung ermöglichen kann, erfolgt üblicherweise parallel zur oder insbesondere entlang der Systemachse.

Gemäß einer Ausführungsform ist das erste Trägerelement relativ zur Aufhängevorrichtung verschiebbar ausgebildet. Typischerweise kann der Abstand zwischen der Aufhängevorrichtung und dem ersten Verbindungselement mittels des relativ zur Aufhängevorrichtung verschiebbar ausgebildeten ersten Trägerelements veränderbar sein. Üblicherweise kann das erste Trägerelement an der Aufhängevorrichtung derart angeordnet sein, dass das erste Trägerelement relativ zur Aufhängevorrichtung verschiebbar insbesondere an einem Aufhängepunkt der Aufhängevorrichtung gelagert ist. Normalerweise kann der Aufhängepunkt dem Drehpunkt um die Systemachse entsprechen. Üblicherweise weist das erste Trägerelement ein erstes Ende und ein zweites Ende auf, wobei das erste Ende und das zweite Ende auf gegenüberliegenden Seiten entlang einer sich in Längsrichtung des ersten Trägerelements erstreckenden Längsachse angeordnet sind. Das zweite Ende des ersten Trägerelements ist üblicherweise mittels des ersten Verbindungselements mit dem zweiten Trägerelement verbunden. Mittels des relativ zur Aufhängevorrichtung verschiebbaren ersten Trägerelements kann das zweite Ende des ersten Trägerelements beispielsweise näher an den Aufhängepunkt verschoben werden, wobei gleichzeitig das erste Ende des ersten Trägerelements von dem Aufhängepunkt entfernt werden kann und umgekehrt.

Wenn das erste Trägerelement relativ zur Aufhängevorrichtung verschiebbar ausgebildet ist, kann auch die Aufhängevorrichtung Mittel aufweisen eine derartige Verschiebung zu ermöglichen. Üblicherweise kann das erste Trägerelement entlang der Längsachse des ersten Trägerelements verschiebbar ausgebildet sein. Typischerweise kann die Verschiebung des relativ zur Aufhängevorrichtung verschiebbar ausgebildeten ersten Trägerelements bedeuten, dass das erste Ende des ersten Trägerelements und das zweite Ende des ersten Trägerelements entlang der Längsachse im gleichen Maße und in der gleichen Richtung verschoben werden können. Dabei kann üblicherweise der Abstand zwischen dem ersten Ende des ersten Trägerelements und dem zweiten Ende des ersten Trägerelements oder die Länge des ersten Trägerelements konstant bleiben. Das erste Trägerelement kann auch derart an der Aufhängevorrichtung angeordnet sein, dass die Längsachse des ersten Trägerelements senkrecht auf der Systemachse steht, wodurch die vorteilhafte horizontale Verschiebung der Haltevorrichtung ermöglicht sein kann.

Gemäß einer weiteren Ausführungsform ist das erste Trägerelement teleskopierbar ausgebildet. Typischerweise kann der Abstand zwischen der Aufhängevorrichtung und dem ersten Verbindungselement auch mittels eines teleskopierbar ausgebildeten ersten Trägerelements veränderbar sein. In diesem Fall kann das zweite Ende des ersten Trägerelements beispielsweise näher an den Aufhängepunkt verschoben werden, wobei das erste Ende des ersten Trägerelements einen konstanten Abstand von dem Aufhängepunkt bewahrt. Es ist denkbar, wenn das erste Trägerelement lediglich teleskopierbar ist, dass das erste Ende des ersten Trägerelements an der Aufhängevorrichtung angeordnet ist. Beispielsweise kann das erste Trägerelement eine Teleskopeinheit aufweisen, welche eingefahren und ausgefahren werden kann. Im Gegensatz zu der Ausführungsform, wenn das erste Trägerelement verschiebbar ausgebildet ist, kann sich der Abstand zwischen den beiden Enden des ersten Trägerelements, wenn die Teleskopeinheit eingefahren oder ausgefahren wird, verändern. Üblicherweise erfolgt dabei eine Veränderung der Länge des ersten Trägerelements. Die beiden Enden des ersten Trägerelements sind üblicherweise weiter voneinander entfernt, wenn die Teleskopeinheit des ersten Trägerelements ausgefahren ist als wenn die Teleskopeinheit des ersten Trägerelements eingefahren ist. Das Einfahren oder das Ausfahren der Teleskopeinheit entspricht einer Teleskopverschiebung. Beim Einfahren kann die Teleskopeinheit üblicherweise kleiner und beim Ausfahren kann die Teleskopeinheit größer werden.

Gemäß einer weiteren Ausführung ist es denkbar, dass das erste Trägerelement teleskopierbar und relativ zur Aufhängevorrichtung verschiebbar ausgebildet ist.

Das zweite Trägerelement ist mit dem ersten Trägerelement mittels des ersten Verbindungselements verbunden, insbesondere derart, dass zwischen dem ersten Trägerelement und dem zweiten Trägerelement ein Winkel ungleich 0 vorliegt. D.h. die Längsachse des ersten Trägerelements und eine sich in Längsrichtung des zweiten Trägerelements erstreckende Längsachse sind insbesondere nicht parallel. Typischerweise kann eine erste Drehebene durch die Längsachse des ersten Trägerelements und durch die Längsachse des zweiten Trägerelements definiert sein.

Gemäß einer weiteren Ausführungsform ist das zweite Trägerelement relativ zum ersten Verbindungselement verschiebbar ausgebildet. Insbesondere das erste Verbindungselement kann Mittel dafür aufweisen, damit das zweite Trägerelement entlang einer sich in Längsrichtung des zweiten Trägerelements erstreckenden Längsachse relativ zum ersten Verbindungselement verschiebbar sein kann. Beispielsweise kann das zweite Trägerelement auf Schienen des ersten Verbindungselements gelagert sein, auf welchen das zweite Trägerelement mittels des ersten Verbindungselements geführt werden kann. Das zweite Trägerelement weist üblicherweise ein erstes Ende und ein zweites Ende auf. Das erste Ende des zweiten Trägerelements und das zweite Ende des zweiten Trägerelements sind dabei insbesondere entlang der Längsachse des zweiten Trägerelements auf gegenüberliegenden Seiten des zweiten Trägerelements angeordnet.

Wenn das erste Ende des zweiten Trägerelements vom ersten Verbindungselement entlang der Längsachse des zweiten Trägerelements weg geschoben wird, kann typischerweise gleichzeitig das zweite Ende des zweien Trägerelements näher an das erste Verbindungselement geschoben werden und umgekehrt. Vorzugsweise kann der Abstand zwischen den beiden Enden des zweiten Trägerelements, insbesondere die Länge des zweiten Trägerelements konstant bleiben.

Der Abstand zwischen dem ersten Verbindungselement und dem zweiten Verbindungselement mittels des zweiten Trägerelements ist gemäß einer weiteren Ausführung veränderbar, wenn das zweite Trägerelement teleskopierbar ausgebildet ist. In diesem Fall weist das zweite Trägerelement beispielsweise eine Teleskopeinheit auf, welche eingefahren und ausgefahren werden kann. Durch das Einfahren oder Ausfahren der Teleskopeinheit kann der Abstand zwischen dem ersten Verbindungselement und dem zweiten Verbindungselement entweder kleiner oder größer werden. Wenn das zweite Trägerelement lediglich teleskopierbar ausgebildet ist, kann gemäß einer weiteren Ausführung das erste Ende des zweiten Trägerelements mit dem ersten Verbindungselement verbunden sein und das zweite Ende des zweiten Trägerelements mit dem zweiten Verbindungselement verbunden sein.

Gemäß einer weiteren Ausführungsform ist das zweite Trägerelement teleskopierbar und relativ zum ersten Verbindungselement verschiebbar ausgebildet.

Vorzugweise ist in einer Ausführung zumindest das erste Trägerelement oder das zweite Trägerelement lediglich teleskopierbar ausgebildet, wodurch eine Kollisionsgefahr beispielsweise eines Benutzers mit dem teleskopierbar ausgebildeten Trägerelement verringert werden kann. Die Kollisionsgefahr beruht üblicherweise darauf, dass die Länge eines Trägerelements konstant bleibt, wenn es lediglich verschiebbar ausgebildet ist. Der Benutzer ist üblicherweise eine Person, welche sich im Untersuchungsraum befindet, beispielsweise ein Patient oder ein Arzt. Mitunter ist es denkbar, dass auch mehrere Benutzer sich im Untersuchungsraum aufhalten können.

In einer weiteren Ausführungsform sind sowohl das erste Trägerelement als auch das zweite Trägerelement lediglich teleskopierbar ausgebildet. Dies ist insofern vorteilhaft, weil dadurch die Kollisionsgefahr der Trägerelemente insbesondere mit dem Benutzer noch kleiner ist, als wenn zumindest ein Trägerelement lediglich verschiebbar ausgebildet ist. Wenn sowohl das erste Trägerelement als auch das zweite Trägerelement lediglich teleskopierbar ausgebildet sind, verändert sich mittels des Einfahren oder des Ausfahren der jeweiligen Teleskopeinheit die Länge des jeweiligen Trägerelements. Ein verschiebbar ausgebildetes Trägerelement hingegen erfordert für einen kollisionsfreien Betrieb Freiraum. Wenn beispielsweise mittels eines relativ zur Aufhängevorrichtung lediglich verschiebbar ausgebildetes erstes Trägerelement der Abstand zwischen der Aufhängevorrichtung und dem ersten Verbindungselement verringert wird, kann in diesen Freiraum das erste Ende des ersten Trägerelements geschoben werden. Außerdem können lediglich teleskopierbare Trägerelemente kompaktere Bauweisen erlauben.

Das zweite Verbindungselement verbindet das zweite Trägerelement und das dritte Trägerelement bevorzugt derart, dass ein Winkel zwischen dem zweiten Trägerelement und dem dritten Trägerelement ungleich 0 vorliegt. D.h. die Längsachse des zweiten Trägerelements und eine sich in Längsrichtung des dritten Trägerelements erstreckende Längsachse sind insbesondere nicht parallel. Typischerweise kann eine zweite Drehebene durch die Längsachse des zweiten Trägerelements und durch die Längsachse des dritten Trägerelements definiert sein.

Gemäß einer weiteren Ausführungsform ist das dritte Trägerelement relativ zum zweiten Verbindungselement um eine sich in Längsrichtung des dritten Trägerelements erstreckende Längsachse drehbar ausgebildet. Üblicherweise kann ein erstes Ende des dritten Trägerelements mit dem zweiten Verbindungselement verbunden sein. Das dritte Trägerelement ist bevorzugt derart ausgebildet, dass an einem zweiten Ende des dritten Trägerelements die Haltevorrichtung verbunden ist. Das erste Ende des dritten Trägerelements und das zweite Ende des dritten Trägerelements liegen üblicherweise auf gegenüberliegenden Seiten des dritten Trägerelements entlang der Längsachse des dritten Trägerelements. Typischerweise ist insbesondere das zweite Ende des dritten Trägerelements relativ zum zweiten Verbindungselement um die Längsachse des dritten Trägerelements drehbar gelagert. Die Längsachse des dritten Trägerelements kann Angularachse genannt sein. Die Angularachse entspricht typischerweise der Drehachse des dritten Trägerelements. Das zweite Ende des dritten Trägerelements kann daher gegenüber dem ersten Ende des dritten Trägerelements verdrehbar sein, wobei das erste Ende des dritten Trägerelements starr bleibt. Daher ist es denkbar, dass das dritte Trägerelement einen Stator und einen Rotor aufweist. Das erste Ende des dritten Trägerelements kann üblicherweise den Stator und das zweite Ende des dritten Trägerelements den Rotor aufweisen. Das dritte Trägerelement kann beispielsweise derart ausgebildet sein, dass der Rotor des dritten Trägerelements relativ zum zweiten Verbindungselement, insbesondere relativ zum Stator des dritten Trägerelements um die Längsachse des dritten Trägerelements drehbar gelagert ist.

Üblicherweise kann der Stator starr mit dem zweiten Verbindungselement verbunden sein.

Grundsätzlich ist es auch in einer weiteren Ausführung denkbar, dass das dritte Trägerelement relativ zum zweiten Verbindungselement verschiebbar und/oder teleskopierbar ausgebildet ist.

Wenn die Haltevorrichtung mit dem zweiten Ende des dritten Trägerelements verbunden ist, kann eine um die Angularachse beschriebene Bewegung der Haltevorrichtung auch als Propellerbewegung bezeichnet werden, weil dann die Haltevorrichtung ähnlich zu einem Propeller eines Flugzeugs drehbar relativ zum zweiten Verbindungselement gelagert sein kann. Dies ist insbesondere bei der Untersuchung eines Patienten vorteilhaft, weil dadurch beispielsweise sowohl die Kollision der Haltevorrichtung mit dem Benutzer als auch mit dem Patienten vermieden werden kann. Insbesondere kann durch eine solche Angularbewegung der Haltevorrichtung die Röntgeneinheit flexibel um den Patienten herum positioniert werden, wobei eine leichtere Durchführung der Bildgebung als auch insbesondere eine höhere Bildqualität erreicht werden kann.

Das Drehgelenk weist üblicherweise geeignete Mittel auf, um die jeweiligen mit dem Drehgelenk verbundenen Trägerelemente zueinander zu verdrehen. Das Drehgelenk kann insbesondere den Winkel zwischen den mit dem Drehgelenk verbundenen Trägerelementen verändern oder lösbar feststellen.

Wenn das erste Verbindungselement das Drehgelenk aufweist, kann das zweite Trägerelement insbesondere um eine erste Drehachse gegenüber dem ersten Trägerelement verdreht werden. Die erste Drehachse erstreckt sich vorzugweise durch das erste Verbindungselement und steht senkrecht auf der ersten Drehebene. Vorzugsweise kann der Winkel zwischen dem ersten Trägerelement und dem zweiten Trägerelement veränderbar sein. Gemäß einer weiteren Ausführung weist lediglich das erste Verbindungselement das Drehgelenk auf.

Wenn das zweite Verbindungselement das Drehgelenk aufweist, kann das dritte Trägerelement insbesondere um eine zweite Drehachse gegenüber dem zweiten Trägerelement verdreht werden. Die zweite Drehachse erstreckt sich vorzugsweise durch das zweite Verbindungselement und steht senkrecht auf der zweiten Drehebene. Vorzugweise kann der Winkel zwischen dem zweiten Trägerelement und dem dritten Trägerelement veränderbar sein. Gemäß einer weiteren Ausführung weist lediglich das zweite Verbindungselement das Drehgelenk auf.

Wenn lediglich das erste Verbindungselement oder lediglich das zweite Verbindungselement das Drehgelenk aufweist oder wenn sowohl das erste Verbindungselement und das zweite Verbindungselement je ein Drehgelenk aufweisen, kann das dritte Trägerelement, insbesondere das zweite Ende des dritten Trägerelements auf der Kreisbahn verfahrbar sein. Des Weiteren kann in diesem Fall das dritte Trägerelement auch höhenverstellbar sein.

Das Röntgensystem kann bevorzugt als Angiographiesystem ausgebildet sein, wobei das Angiographiesystem bevorzugt für die Durchführung einer angiographischen Messung geeignet ist.

Die Haltevorrichtung kann insbesondere mit dem dritten Trägerelement mittels der Verbindungsvorrichtung verbunden sein. Vorzugsweise kann gemäß einer Ausführung die Verbindungsvorrichtung um die Angularachse, welche der Längsachse des dritten Trägerelements entspricht, gedreht werden. Wenn die Verbindungsvorrichtung um die Angularachse gedreht werden kann, kann normalerweise die Haltevorrichtung und insbesondere der C-Bogen gleichzeitig um die Angularachse gedreht werden. Beispielsweise kann die Verbindungsvorrichtung einen Aufhängepunkt aufweisen, an welchem die Verbindungsvorrichtung, insbesondere die Haltevorrichtung mit dem dritten Trägerelement verbunden ist. Vorzugweise kann der Aufhängepunkt der Verbindungsvorrichtung an dem zweiten Ende des dritten Trägerelements angeordnet sein. Die Anordnung des Aufhängepunkts der Verbindungsvorrichtung an dem zweiten Ende des dritten Trägerelements kann einer Befestigung entsprechen.

Der C-Bogen kann üblicherweise die Positionierung eines Untersuchungsbereichs in einem Patienten auf einer imaginären Linie zwischen den beiden Enden des C-Bogens ermöglichen. Der C-Bogen kann üblicherweise die Röntgeneinheit aufweisen, wobei der Röntgenstrahler an einem ersten Ende des C-Bogens und der Röntgendetektor an einem zweiten Ende des C-Bogens angeordnet sind. Typischerweise weist der C-Bogen insbesondere eine kreisbogenförmige Form auf. Alternativ ist es auch denkbar, dass der C-Bogen eine andere Form, beispielsweise eine teilweise eckige Form, möglicherweise eine Form eines offenen halben Rechtecks, aufweist. Die kreisbogenförmige Ausführung des C-Bogen kann insbesondere vorteilhaft sein im Vergleich zu einem anderen als ein offenes halbes Rechteck ausgebildeten C-Bogen, weil die kreisbogenförmige Ausführung des C-Bogens die Kollisionsgefahr mit einem Patienten verringern kann.

Eine Orbitalachse kann insbesondere senkrecht auf einer durch die Form des C-Bogens definierten C-Bogen-Ebene stehen. Außerdem kann üblicherweise durch die Form des C-Bogens die erste Bahn vorgegeben sein, auf der die Röntgeneinheit relativ zur Verbindungsvorrichtung verfahrbar angeordnet sein kann. Das Verfahren der Röntgeneinheit in der C-Bogen-Ebene kann einer Orbitalbewegung entsprechen. Daher kann die Röntgeneinheit, insbesondere der Röntgenstrahler und der Röntgendetektor in der C-Bogen-Ebene um eine orbitale Drehachse entlang eines orbitalen Drehkreises verfahren werden. Die orbitale Drehachse entspricht üblicherweise der Orbitalachse. Vorzugsweise ist die Röntgeneinheit, insbesondere sind der Röntgenstrahler und der Röntgendetektor entlang der ersten Bahn mit einer Bogenlänge verfahrbar, wobei ein erster Drehkreis die erste Bahn aufweisen kann. Jede Bogenlänge ist in einem Kreis mit einem jeweiligen Radius enthalten. Die Bogenlänge beispielsweise der ersten Bahn kann üblicherweise mittels eines Radius des jeweiligen Kreises, der die erste Bahn aufweist, in einen ersten Winkel umgerechnet werden. Die Bogenlänge der ersten Bahn entspricht daher dem ersten Winkel.

Vorzugsweise kann die Haltevorrichtung ausschließlich mittels einer Bewegung des C-Bogens relativ zur Verbindungsvorrichtung auf der ersten Bahn verfahren werden. Dafür kann die Verbindungsvorrichtung geeignete Mittel, beispielsweise einen Laufwagen aufweisen, der mit dem C-Bogen derart verbunden ist, dass der C-Bogen relativ zur Verbindungsvorrichtung verfahrbar ist. Beispielsweise kann der C-Bogen Rollen aufweisen, die auf Schienen des Laufwagens geführt werden. Wenn die Röntgeneinheit gegenüber dem Laufwagen verschoben werden kann, kann dies insbesondere einer Verschiebung der Röntgeneinheit relativ zur Verbindungsvorrichtung entsprechen. Die Verbindungsvorrichtung kann auch weitere Mittel aufweisen, die eine Verschiebung der Röntgeneinheit relativ zur Verbindungsvorrichtung ermöglichen können. Üblicherweise ist auch die Röntgeneinheit dementsprechend ausgebildet. Alternativ oder zusätzlich zu dem Laufwagen kann die Verbindungsvorrichtung eine dritte Schienenebene aufweisen, wobei der C-Bogen auf den Schienen der dritten Schienenebene verfahrbar gelagert ist. Die dritte Schienenebene kann das Verfahren des C-Bogens, insbesondere des Röntgenstrahlers und des Röntgendetektors entlang der ersten Bahn ermöglichen.

Gemäß einer geeigneten Ausführung kann die Tragevorrichtung eine Bewegung des dritten Trägerelements entlang der Kreisbahn durchführen, wobei vorzugweise ein erster Drehkreis die Kreisbahn aufweist. Die Haltevorrichtung kann relativ zur Verbindungsvorrichtung auf einem zweiten Drehkreis verschoben werden. Bevorzugt ist insbesondere eine Ausführung, bei welcher der erste Drehkreis dem zweiten Drehkreis und auch dem orbitalen Drehkreis entspricht. Vorzugsweise sind der erste Drehkreis, der zweite Drehkreis und der orbitale Drehkreis deckungsgleich zu dem Drehkreis. Die zweite Bahn kann insofern Teil des Drehkreises sein, wenn die zweite Bahn in dem orbitalen Drehkreis enthalten ist. Insbesondere kann die Bogenlänge der zweiten Bahn deckungsgleich sein mit einem Abschnitt der Kreisbahn des Drehkreises.

Die Röntgeneinheit kann üblicherweise auf dem orbitalen Drehkreis zwischen einer ersten Extremposition und einer zweiten Extremposition verfahrbar sein. Die Bewegung zwischen der ersten Extremposition und der zweiten Extremposition entlang des Drehkreises, insbesondere des orbitalen Drehkreises erfolgt mittels einer Konfiguration der Tragevorrichtung gemeinsam mit der relativ zur Verbindungsvorrichtung verfahrbaren Röntgeneinheit. Der Röntgenstrahler und der Röntgendetektor sind mittels des ersten Trägerelements, des Drehgelenks, des zweiten Trägerelements und der Bewegung des C-Bogens relativ zur Verbindungsvorrichtung entlang einer zweiten Bahn, die Teil des Drehkreises ist und deren Bogenlänge einem zweiten Winkel entspricht, verfahrbar angeordnet, wobei der zweite Winkel größer ist als der erste Winkel. Beispielsweise sind durch den zweiten Winkel die erste Extremposition und die zweite Extremposition des Röntgenstrahlers und des Röntgendetektors definiert.

Gemäß einer Ausführungsform beträgt der zweite Winkel mindestens 200°, was üblicherweise für eine dreidimensionale (3D) Bildgebung vorteilhaft ist. Derart kann die Orbitalbewegung über eine Bogenlänge, die mindestens einem Winkel von 200° entspricht, möglich sein. Die Orbitalbewegung um mindestens 200° ist typischerweise notwendig, damit die Bildrekonstruktion bei der dreidimensionalen Bildgebung bei der Durchführung einer angiographischen Messung des Patienten funktioniert. Aufgrund der Kollisionsgefahr zwischen der Haltevorrichtung und des Patienten bzw. der bauartbedingt großen Ausführung werden üblicherweise keine C-Bögen mit einer Bogenlänge, die einem Winkel größer als 180° entsprechen, eingesetzt. Daher kann üblicherweise der C-Bogen relativ zu der Verbindungsvorrichtung verfahrbar gelagert sein.
Aufgrund der Aufbauten wie Röntgenstrahler und -detektor ist in herkömmlichen Röntgensystemen typischerweise eine mehrstufige Haltevorrichtung zur Ausführung der Orbitalbewegung nötig. Eine derartige mehrstufige Haltevorrichtung in herkömmlichen Röntgensystemen weist beispielsweise einen Laufwagen auf, der einen weiteren auf einer Kreisbahn verfahrbaren Teleskopwagen umfasst, worin der auf einem Rollenlager verfahrbar gelagerte C-Bogen geführt werden kann. Daraus folgt, dass die derartige mehrstufige Haltevorrichtung in herkömmlichen Röntgensystemen üblicherweise eine orbitale Teleskopeinheit aufweist, welche im Gegensatz zu der Teleskopeinheit der Trägerelemente nicht entlang einer geraden Linie, sondern auf einer Kreisbahn verfahrbar ist. In diesem Zusammenhang weist eine derartige Haltevorrichtung neben dem C-Bogen noch einen G-Bogen auf, wobei die orbitale Teleskopeinheit üblicherweise den C-Bogen und den G-Bogen aufweist. Der G-Bogen kann relativ zum C-Bogen verfahrbar und der C-Bogen relativ zu dem Laufwagen verfahrbar angeordnet sein. Die derartige mehrstufige Haltevorrichtung greift oft auf eine Ausführung mit dem G-Bogen und dem C-Bogen als Teil der orbitalen Teleskopeinheit zurück. Daher kann eine derartige mehrstufige Haltevorrichtung die Orbitalbewegung der Röntgeneinheit um 200° ausführen.

Gemäß einer erfindungsgemäßen Ausführung wird die Haltevorrichtung insbesondere keine orbitale Teleskopeinheit aufweisen, sondern die Tragevorrichtung kann zusammen mit der Haltevorrichtung die Orbitalbewegung der Röntgeneinheit mindestens um 200° durchführen. Daher ist das Röntgensystem vorteilhafterweise bauartbedingt kleiner im Vergleich zu einem anderen Röntgensystem mit einer mehrstufigen Haltevorrichtung, wodurch beispielsweise das Röntgensystem auch in kleineren Untersuchungszimmern genutzt werden kann.

Gemäß einer weiteren Ausführung ist die Haltevorrichtung entlang der Systemachse mittels des ersten Trägerelements, des Drehgelenks und des zweiten Trägerelements höhenverstellbar. Die Tragevorrichtung kann derart ausgebildet sein, dass die Haltevorrichtung lediglich entlang der Systemachse oder parallel zur Systemachse höhenverstellbar ist. Vorzugsweise können auch nur bestimmte Komponenten der Haltevorrichtung höhenverstellbar sein, beispielsweise die Röntgeneinheit. Zum Verfahren von einer niedrigeren zu einer höheren Position der Röntgeneinheit kann beispielsweise der Abstand zwischen dem ersten Verbindungselement und der Aufhängevorrichtung vergrößert werden, während der Abstand zwischen dem ersten Verbindungselement und dem zweiten Verbindungselement verringert werden kann. Beispielsweise kann auch lediglich mittels Veränderung des Abstands zwischen dem ersten Verbindungselement und dem zweiten Verbindungselement das Röntgensystem höhenverstellbar sein, wenn vorzugweise die Längsachse des zweiten Trägerelements parallel zur Systemachse ist. Der jeweilige Abstand kann je nach Ausführung der Trägerelemente unterschiedlich veränderbar sein: beispielsweise mittels einer Teleskopverschiebung der Teleskopeinheit oder mittels einer Verschiebung des jeweiligen Trägerelements bezogen auf den jeweiligen Aufhängepunkt, d.h. beispielsweiser mittels einer Verschiebung des ersten Trägerelements relativ zur Aufhängevorrichtung oder insbesondere mittels einer Verschiebung des zweiten Trägerelements relativ zum ersten Verbindungselement. Eine Höhenverstellbarkeit der Haltevorrichtung ist insbesondere vorteilhaft, wenn die Patientenliege höhenverstellbar ist und der Benutzer die Patientenliege und die Haltevorrichtung beispielsweise in der Höhe aufeinander abstimmen kann. Dies kommt insbesondere der Gesundheit des Benutzers zu Gute oder ist auch beispielsweise bei der Durchführung eines interventionellen Eingriffs vorteilhaft, welcher auf der Patientenliege vor, nach oder während einer angiographischen Messung durchgeführt werden kann.

Gemäß einer Ausführungsform ist der Abstand zwischen dem Röntgenstrahler und dem Röntgendetektor veränderbar. Der Abstand zwischen dem Röntgenstrahler und dem Röntgendetektor kann derart veränderbar sein, dass lediglich mittels einer geeigneten Konfiguration der Haltevorrichtung der Abstand zwischen dem Röntgenstrahler und dem Röntgendetektor variiert werden kann. Üblicherweise kann der Röntgendetektor eine Teleskopeinheit aufweisen, welche eingefahren und ausgefahren werden kann. Dies dient üblicherweise der Fokussierung der Röntgenstrahlung und/oder insbesondere der Ausrichtung des Isozentrums. Insbesondere kann der Röntgendetektor so gelagert sein, dass der Röntgendetektor um eine Achse, die einer Linie zwischen dem Röntgenstrahler und dem Röntgendetektor entspricht und üblicherweise senkrecht auf der Röntgendetektoroberfläche steht, drehbar ist.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine erste Ausführungsform einer Tragevorrichtung,
- Fig. 2: eine zweite Ausführungsform einer Tragevorrichtung,
- Fig. 3: eine Ausführungsform eines Röntgensystems,
- Fig. 4: eine erste Extremposition des Röntgensystems,
- Fig. 5: eine zweite Extremposition des Röntgensystems und
- Fig. 6: einen Drehkreis.

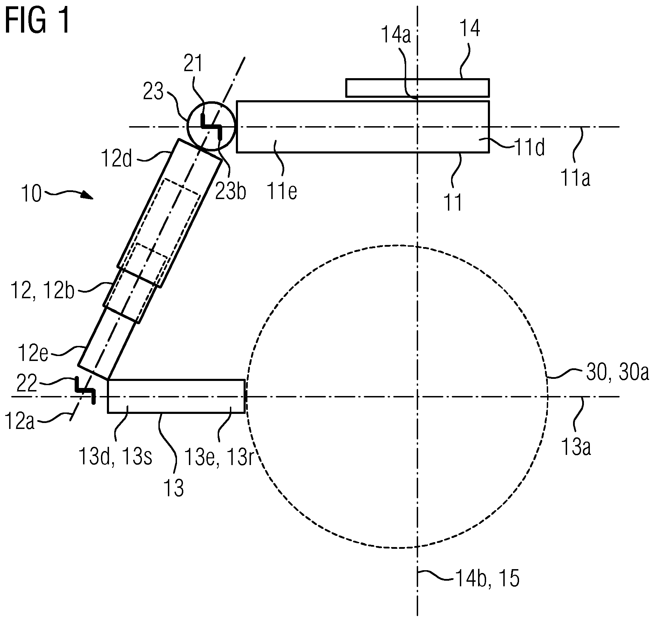

Fig. 1 zeigt eine erste Ausführungsform einer Tragevorrichtung 10. Die Tragevorrichtung 10 für eine Haltevorrichtung für einen Röntgenstrahler und einen Röntgendetektor eines Röntgensystems weist eine Aufhängevorrichtung 14, ein an der Aufhängevorrichtung 14 angeordnetes erstes Trägerelement 11, ein zweites Trägerelement 12 und ein erstes Verbindungselement 21 auf. Das zweite Trägerelement 12 ist mittels des ersten Verbindungselements 21 mit dem ersten Trägerelement 11 verbunden. Weiterhin weist die Tragevorrichtung 10 ein drittes Trägerelement 13, das mit der Haltevorrichtung verbindbar ist, und ein zweites Verbindungselement 22 auf, wobei das dritte Trägerelement 13 mittels des zweiten Verbindungselements 22 mit dem zweiten Trägerelement 12 verbunden ist. Im in Fig. 1 gezeigten Fall weist das erste Verbindungselement 21 ein Drehgelenk 23 auf.

Der Abstand zwischen der Aufhängevorrichtung 14 und dem ersten Verbindungselement 21 ist mittels des ersten Trägerelements 11 veränderbar und der Abstand zwischen dem ersten Verbindungselement 21 und dem zweiten Verbindungselement 22 ist mittels des zweiten Trägerelements 12 veränderbar.

Das erste Trägerelement 11 ist relativ zur Aufhängevorrichtung 14 verschiebbar und das zweite Trägerelement 12 ist teleskopierbar ausgebildet. Weitere Ausführungen sind denkbar, beispielsweise, dass das erste Trägerelement 11 teleskopierbar und das zweite Trägerelement 12 relativ zum ersten Verbindungselement 21 verschiebbar ausgebildet ist. Es ist auch denkbar, dass das erste Trägerelement 11 und das zweite Trägerelement 12 teleskopierbar ausgebildet sind.

Die Aufhängevorrichtung 14 ist in diesem Beispiel für eine Anordnung des ersten Trägerelements 11 an einer Decke des Untersuchungsraums ausgebildet. Die Tragevorrichtung 10 weist eine Systemachse 15 auf, welche bei einer Aufhängung der Tragevorrichtung 10 an der Decke oder dem Boden senkrecht auf der Decke oder dem Boden steht und durch die Aufhängevorrichtung 14 verläuft. Das erste Trägerelement 11 ist an der Aufhängevorrichtung 14 drehbar um die Systemachse 15 gelagert. Das erste Trägerelement kann auch um eine Drehachse 14b der Aufhängevorrichtung 14 drehbar gelagert sein. Die Drehachse 14b der Aufhängevorrichtung 14 entspricht in dieser Ausführung der Systemachse 15. Die Systemachse 15 verläuft durch einen Aufhängepunkt 14a und ist vertikal ausgerichtet. Am Aufhängepunkt 14a ist das erste Trägerelement 11 mit einem Aufhängungsmittel, beispielsweise auf Rollen oder Schienen gelagert oder insbesondere befestigt, angeordnet. Die Aufhängevorrichtung 14 ist längs der Systemachse 15 kleiner dimensioniert als senkrecht zur Systemachse 15. Die Aufhängevorrichtung 14 ist insbesondere flach ausgestaltet.
Das erste Trägerelement 11 weist eine sich in Längsrichtung des ersten Trägerelements 11 erstreckende Längsachse 11a, welche die Achse der Verschiebung des ersten Trägerelements 11 relativ zur Aufhängevorrichtung 14 darstellt, auf. Weiterhin weist das erste Trägerelement ein erstes Ende 11d des ersten Trägerelements 11 und ein zweites Ende 11e des ersten Trägerelements 11 auf. Die Längsachse 11a des ersten Trägerelements 11 steht senkrecht auf der Systemachse 15 und ist demnach horizontal ausgerichtet. Das erste Trägerelement 11 kann den Abstand zwischen der Aufhängervorrichtung 14, insbesondere den Aufhängepunkt 14a und dem ersten Verbindungselement 21 verändern. Beispielsweise wird, wenn der Abstand zwischen der Aufhängevorrichtung 14 und dem ersten Verbindungselement 21 kleiner wird, gleichzeitig der Abstand zwischen der Aufhängevorrichtung 14 und dem ersten Ende 11d des ersten Trägerelements 11 größer. Das erste Trägerelement 11 wird also entlang der Längsachse 11a des ersten Trägerelements 11 relativ zur Aufhängevorrichtung 14 verschoben, ohne dass der Abstand zwischen dem ersten Ende 11d des ersten Trägerelements 11 und dem zweiten Ende 11e des ersten Trägerelements 11 sich verändert. Wenn das erste Trägerelement 11 entlang der Längsachse 11a des ersten Trägerelements 11 verschoben wird, werden unter anderem gleichzeitig das erste Verbindungselement 21, das zweite Trägerelement 12, das zweite Verbindungselement 22 und das dritte Trägerelement 13 im gleichen Maße entlang der Längsachse 11a verschoben. Wenn das erste Trägerelement 11 relativ zur Aufhängevorrichtung 14 verschoben wird, wird auch die mit dem dritten Trägerelement 13 verbindbare Haltevorrichtung verschoben. Dabei bleibt die Ausdehnung des ersten Trägerelements 11 in Längsrichtung 11a, sprich die Länge des ersten Trägerelements 11 konstant.

Das zweite Trägerelement 12 weist eine sich in Längsrichtung des zweiten Trägerelements 12 erstreckende Längsachse 12a auf, entlang welcher der Abstand zwischen dem ersten Verbindungselement 21 und dem zweiten Verbindungselement 22 verändert werden kann. Weiterhin weist das zweite Trägerelement 12 ein erstes Ende 12d des zweiten Trägerelements 12 und ein zweites Ende 12e des zweiten Trägerelements 12 auf. Das zweite Trägerelement 12 weist eine Teleskopeinheit 12b auf, welche eingefahren und ausgefahren werden kann. Wenn die Teleskopeinheit 12b eingefahren wird, verringert sich der Abstand zwischen dem ersten Ende 12d des zweiten Trägerelements 12 und dem zweiten Ende 12e des zweiten Trägerelements 12, dementsprechend verringert sich auch der Abstand zwischen dem ersten Verbindungselement 21 und dem zweiten Verbindungselement 22. Das Verbindungselement 22 und damit auch das Trägerelement 13 werden also entlang der Längsachse 12a relativ zum Verbindungselement 21 mittels des zweiten Trägerelements 12, insbesondere mittels der Teleskopeinheit 12b des zweiten Trägerelements 12, verschoben. Bei der Teleskopverschiebung mittels der Teleskopeinheit 12b verändert sich die Ausdehnung des zweiten Trägerelements 12 in Längsrichtung 12a, sprich die Länge des zweiten Trägerelements 12.

Das dritte Trägerelement 13 weist eine sich in Längsrichtung des dritten Trägerelements 13 erstreckende Längsachse 13a, ein erstes Ende 13d des dritten Trägerelements 13 und ein zweites Ende 13e des dritten Trägerelements 13 auf. Das dritte Trägerelement 13 ist relativ zum zweiten Verbindungselement 22 um die Längsachse 13a drehbar ausgebildet. Das zweite Ende 13e des dritten Trägerelements 13 kann insbesondere gegenüber dem ersten Ende 13d des dritten Trägerelements 13 um die Längsachse 13a des dritten Trägerelements verdrehbar sein. In diesem Fall ist also nur das zweite Ende 13e des dritten Trägerelements 13 relativ zum zweiten Verbindungselement 22 verdrehbar, während das erste Ende 13d des dritten Trägerelements 13 nicht relativ zum zweiten Verbindungselement 22 verdrehbar ist. Das erste Ende 13d weist dafür einen Stator 13s auf, während das zweite Ende 13e einen Rotor 13r aufweist. Im in Fig. 1 gezeigten Fall ist also nur ein Teil des dritten Trägerelements 13 relativ zum zweiten Verbindungselement 22 um die sich in Längsrichtung des dritten Trägerelements 13 erstreckende Längsachse 13a drehbar ausgebildet. Es ist auch der alternative Fall denkbar, dass das gesamte dritte Trägerelement 13 relativ zum zweiten Verbindungselement 22 um die sich in Längsrichtung des dritten Trägerelements 13 erstreckende Längsachse 13a drehbar ausgebildet ist. Grundsätzlich ist es in einer anderen Ausführung auch denkbar, dass der Abstand zwischen dem zweiten Verbindungselement 22 und der am dritten Trägerelement 13 verbindbaren Haltevorrichtung veränderbar ist. Beispielsweise kann das dritte Trägerelement 13 teleskopierbar und/oder relativ zum zweiten Verbindungselement 22 verschiebbar ausgebildet sein.

Das erste Trägerelement 11, das zweite Trägerelement 12 und das dritte Trägerelement 13 sind stabförmig entlang der jeweiligen Längsrichtungen 11a, 12a und 13a ausgebildet. Beispielsweise können aber das erste Trägerelement 11, das zweite Trägerelement und das dritte Trägerelement 13 unabhängig voneinander unterschiedliche Formen annehmen, insbesondere können das erste Trägerelement 11, das zweite Trägerelement 12 und das dritte Trägerelement 13 einen Knick oder eine L-Form oder eine weitere geeignete Form aufweisen.

Das zweite Trägerelement 12 ist mittels des ersten Verbindungselements 21 in einem Winkel ungleich 0 und kleiner als 180° mit dem ersten Trägerelement 11 verbunden. Der Winkel kann dabei mittels einer Drehung des zweiten Trägerelements 12 gegenüber dem ersten Trägerelement 11 mittels des Drehgelenks 23 verstellt werden. Die Längsachse 11a des ersten Trägerelements 11 und die Längsachse 12a des zweiten Trägerelements 12 bilden also eine erste Ebene. Weil das erste Verbindungselement 21 das Drehgelenk 23 aufweist, ist die erste Ebene identisch zu einer ersten Drehebene des Drehgelenks 23. Das zweite Trägerelement 12 kann also innerhalb der ersten Drehebene des Drehgelenks 23 mittels des Drehgelenks 23 relativ zum ersten Trägerelement 11 um eine Drehachse 23b verdreht werden. Die Drehachse 23b ist der Schnittpunkt der Längsachse 11a des ersten Trägerelements 11 und der Längsachse 12a des zweiten Trägerelements 12b. Der Winkel zwischen dem ersten Trägerelement 11 und dem zweiten Trägerelement 12 ist somit veränderbar.
Das dritte Trägerelement 13 ist mittels des zweiten Verbindungselements 22 in einem Winkel ungleich 0 und kleiner als 180° mit dem zweiten Trägerelement 12 verbunden. Der Winkel zwischen dem zweiten Trägerelement 12 und dem dritten Trägerelement 13 ist konstant. Die Längsachse 12a des zweiten Trägerelements 12 und die Längsachse 13a des dritten Trägerelements 13 bilden also eine zweite Ebene, wobei die zweite Ebene identisch zur ersten Ebene ist. In der ersten Drehebene des Drehgelenks 23 liegen also die Längsachse 11a des ersten Trägerelements 11, die Längsachse 12a des zweiten Trägerelements 12 und die Längsachse 13a des dritten Trägerelements 13. Die Systemachse 15 ist in der ersten Drehebene des Drehgelenks 23 enthalten. Dadurch ist es möglich, dass das dritte Trägerelement 13, insbesondere das zweite Ende 13e des dritten Trägerelements parallel zur Systemachse 15 höhenverstellbar ist.

Das erste Verbindungselement 21 und das zweite Verbindungselement 22 weisen Mittel zur Verbindung der jeweiligen Trägerelemente 11, 12, 13 auf.

Das Drehgelenk 23 ermöglicht das Verändern der Winkel zwischen dem ersten Trägerelement 11 und dem zweiten Trägerelement 12. Das dritte Trägerelement 13, insbesondere das zweite Ende 13e des dritten Trägerelements 13, ist somit vorteilhafterweise mittels des ersten Trägerelements 11, des Drehgelenks 23 und des zweiten Trägerelements 12 entlang einer Kreisbahn 30a, die Teil eines Drehkreises 30 ist, verfahrbar angeordnet.

Beispielsweise kann das zweite Ende 13e entlang des Drehkreises 30 verfahren werden, wenn der Abstand zwischen der Aufhängevorrichtung 14 und dem ersten Verbindungselement 21 und der Abstand zwischen dem ersten Verbindungselement 21 und dem zweiten Verbindungselement 22 größer werden und gleichzeitig der Winkel zwischen dem ersten Trägerelement 11 und dem zweiten Trägerelement 12 kleiner wird.

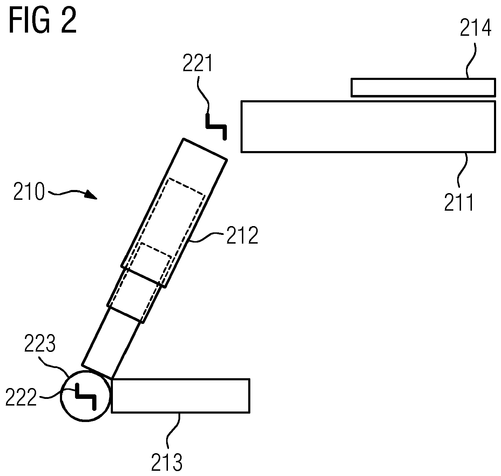

Fig. 2 zeigt eine zweite Ausführungsform einer Tragevorrichtung 210. In Fig. 2 sind aus Gründen der Übersichtlichkeit lediglich die wesentlichen Unterschiede mit Bezugszeichen im Vergleich zur in Fig. 1 gezeigten ersten Ausführungsform dargestellt. Die nachfolgenden Beschreibungen beschränken sich daher im Wesentlichen auf die Unterschiede zu dem Ausführungsbeispiel der Fig. 1.

Die Tragevorrichtung 210 für eine Haltevorrichtung für einen Röntgenstrahler und einen Röntgendetektor eines Röntgensystems weist eine Aufhängevorrichtung 214, ein an der Aufhängevorrichtung 214 angeordnetes erstes Trägerelement 211, ein zweites Trägerelement 212 und ein erstes Verbindungselement 221 auf. Das zweite Trägerelement 212 ist mittels des ersten Verbindungselements 221 mit dem ersten Trägerelement 211 verbunden. Weiterhin weist die Tragevorrichtung 210 ein drittes Trägerelement 213, das mit der Haltevorrichtung verbindbar ist, und ein zweites Verbindungselement 222 auf, wobei das dritte Trägerelement 213 mittels des zweiten Verbindungselements 222 mit dem zweiten Trägerelement 212 verbunden ist.

Der Unterschied zu Fig. 1 ist, dass das zweite Verbindungselement 222 das Drehgelenk 223 aufweist, während in Fig. 1 das erste Verbindungselement 21 das Drehgelenk 23 aufweist. Damit ist die Veränderung eines Winkels zwischen dem zweiten Trägerelement 212 und dem dritten Trägerelement 213 möglich in einer zweiten Drehebene des Drehgelenks 223 möglich. Die zweite Drehebene ist gebildet durch eine sich in Längsrichtung des zweiten Trägerelements 212 erstreckende Längsachse und eine sich in Längsrichtung des dritten Trägerelements 213 erstreckende Längsachse. Auf der zweiten Drehebene steht eine zweite Drehachse des Drehgelenks 223 senkrecht. Das dritte Trägerelement 213 kann um die zweite Drehachse des Drehgelenks 223 relativ zum zweiten Trägerelement 212 drehbar sein. Die Tragevorrichtung 210 der Fig. 2 weist im Wesentlichen die gleichen Funktionen und Eigenschaften auf wie die Tragevorrichtung 10 aus Fig. 1.

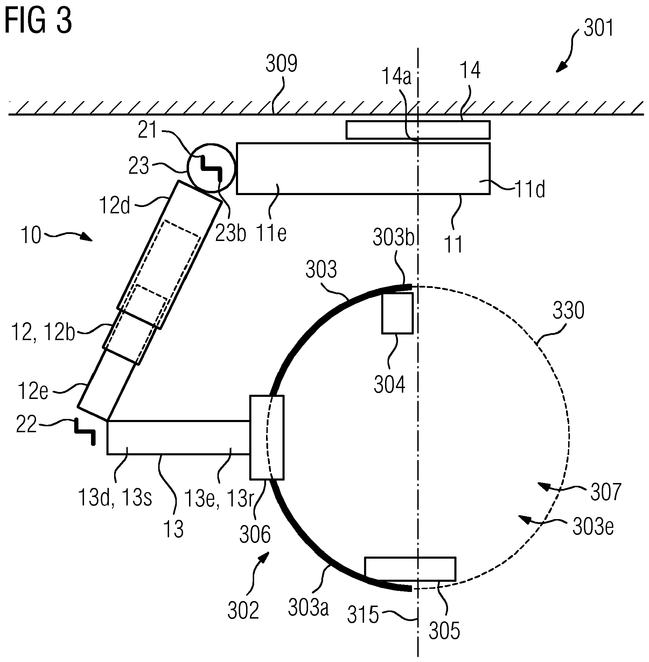

Fig. 3 zeigt eine Ausführungsform eines Röntgensystems 301. Die in Fig. 3 gezeigte Ausführungsform des Röntgensystems 301 weist die Tragevorrichtung 10 gemäß Fig. 1 auf, so dass auf eine erneute Beschreibung der Tragevorrichtung 10 verzichtet wird.

Das Röntgensystem 301 weist neben der Tragevorrichtung 10 eine mit dem dritten Trägerelement 13 der Tragevorrichtung 10 verbundene Haltevorrichtung 302 auf. Die Haltevorrichtung 302 weist einen C-Bogen 303, an dessen Enden ein Röntgenstrahler 304 und ein Röntgendetektor 305 angeordnet sind, und eine Verbindungsvorrichtung 306 auf. Der Röntgenstrahler 304 und der Röntgendetektor 305 sind entlang einer von der Form des C-Bogens 303 vorgegebenen ersten Bahn 630a, die Teil eines Drehkreises 330 ist und deren Bogenlänge einem ersten Winkel entspricht, ausschließlich mittels einer Bewegung des C-Bogens 303 relativ zur Verbindungsvorrichtung 306 verfahrbar angeordnet. Ein erstes Ende 303a des C-Bogens 303 weist den Röntgendetektor 305 auf und ein zweites Ende 303b des C-Bogens 303 weist den Röntgenstrahler 304 auf. Der Drehkreis 330 befindet sich in einer von der Form des C-Bogens 303 vorgegebenen C-Bogen-Ebene 303e. Der Drehkreis 330 entspricht einem orbitalen Drehkreis der Haltevorrichtung 302, entlang welchem eine Orbitalbewegung der Haltevorrichtung 302 möglich ist.

Das Röntgensystem 301 weist eine Systemachse 315 auf, welche bei einer Aufhängung der Tragevorrichtung 10 an einer Decke 309 senkrecht auf der Decke 309 steht und durch die Aufhängevorrichtung 14 verläuft, wobei die Haltevorrichtung 302 entlang der Systemachse 315 mittels der Tragevorrichtung 10 höhenverstellbar ist. Die Systemachse 315 des Röntgensystems 301 ist identisch zur Systemachse 15 der Tragevorrichtung 10. Die Haltevorrichtung 302 ist also entlang der Systemachse 315 mittels des ersten Trägerelements 11, des Drehgelenks 23 und des zweiten Trägerelements 12 höhenverstellbar.

Das Röntgensystem 301 ist mittels der Aufhängevorrichtung 14 an der Decke 309 angeordnet, insbesondere derart befestigt, dass die Tragevorrichtung 10 an der Decke 309 hängt. Die Aufhängevorrichtung 14 ist weiterhin auf nicht gezeigten Schienen einer ersten Schienenebene und auf nicht gezeigten Schienen einer zweiten Schienenebene verfahrbar gelagert. Die sich in Längsrichtung der Schienen der ersten Schienenebene und der zweiten Schienenebene erstreckenden Längsachsen stehen aufeinander senkrecht. Die erste Schienenebene und die zweite Schienenebene sind planparallel zur Decke 209 und die Systemachse 315 des Röntgensystems 301 steht darauf senkrecht.

Eine Röntgeneinheit 307 weist den Röntgenstrahler 304 und den Röntgendetektor 305 auf. Der Abstand zwischen dem Röntgenstrahler 304 und dem Röntgendetektor 305 ist veränderbar, beispielsweise wenn die Röntgeneinheit eine Teleskopeinheit aufweist. Nicht gezeigt ist eine höhenverstellbare Patientenliege. Durch die Höhenverstellbarkeit des Röntgensystems 301 und der höhenverstellbaren Patientenliege kann ein Benutzer eine optimale Position und Ausrichtung des Röntgensystems 301 und der höhenverstellbaren Patientenliege für die Behandlung eines auf der Patientenliege positionierten Patienten wählen.

Die Haltevorrichtung 302 ist derart mit dem dritten Trägerelement 13 verbunden, dass das dritte Trägerelement 13, insbesondere das zweite Ende 13e des dritten Trägerelements 13 gemeinsam mit der Haltevorrichtung 302 im Untersuchungsraum verfahrbar ist. Beispielsweise folgt aus der Höhenverstellbarkeit des dritten Trägerelements 13 mittels der Tragevorrichtung 10 eine Höhenverstellbarkeit der Haltevorrichtung 302 mittels der Tragevorrichtung 10. In einer weiteren Ausführung kann die Haltevorrichtung 302 analog zum zweiten Ende 13e des dritten Trägerelements 13 relativ zum zweiten Verbindungselement 22 um die Längsachse 13a des dritten Trägerelements 13 verdreht werden.

Besonders vorteilhafte Konfigurationen des Röntgensystems 301 bedingen sowohl eine bestimmte Konfiguration der Tragevorrichtung 10, als auch gleichzeitig eine bestimmte Konfiguration der Haltevorrichtung 302. In Fig. 3 entspricht der Drehkreis 330 der Haltevorrichtung 302 dem Drehkreis 30 des dritten Trägerelements 13. Beispielsweise kann dadurch die Haltevorrichtung 302 auf demselben Drehkreis 330 verfahren werden wie das dritte Trägerelement 13. Das Röntgensystem 301 ermöglicht dies sowohl durch eine bestimmte Konfiguration der Tragevorrichtung 10, als auch durch eine andere Konfiguration der Haltevorrichtung 302. Im letzteren Fall können beispielsweise ohne Bewegung des ersten Trägerelements 11, des Drehgelenks 23 und des zweiten Trägerelements 12 der Röntgenstrahler 304 und der Röntgendetektor 305 auf dem Drehkreis 330 verfahren werden.

Folgende Bewegungsmöglichkeiten sind mittels des in Fig. 3 gezeigten Röntgensystems 301 möglich:
- eine Bewegung der Tragevorrichtung 10 in Längsrichtung der höhenverstellbaren Patientenliege mittels der Aufhängevorrichtung 14,
- eine Bewegung der Tragevorrichtung 10 senkrecht zur Längsrichtung der höhenverstellbaren Patientenliege mittels der Aufhängevorrichtung 14,
- eine Drehbewegung der Tragevorrichtung 10 um die Systemachse 15 um bis zu +/- 135°,
- eine Drehung der Haltevorrichtung 302 um die Angularachse, die der Längsachse 13a entspricht, im Bereich von -190° bis zu +120°,
- eine Orbitalbewegung der Röntgeneinheit 307 entlang des Drehkreises 330 um mindestens 200°,
- eine Veränderung des Abstands zwischen dem Röntgenstrahler 304 und dem Röntgendetektor von 900 bis 1300mm und
- eine Drehung des Röntgendetektors 305 um eine nicht gezeigte Röntgendetektorachse, die senkrecht auf der Röntgendetektoroberfläche steht, um bis zu 360°.

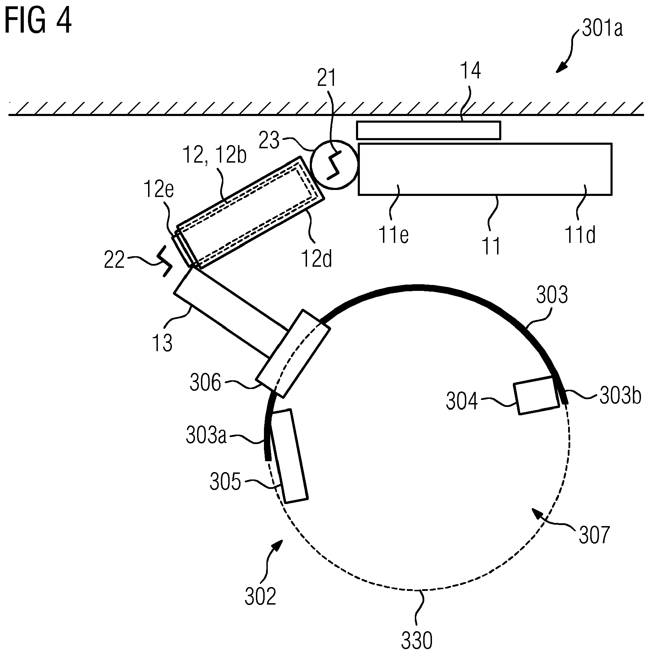

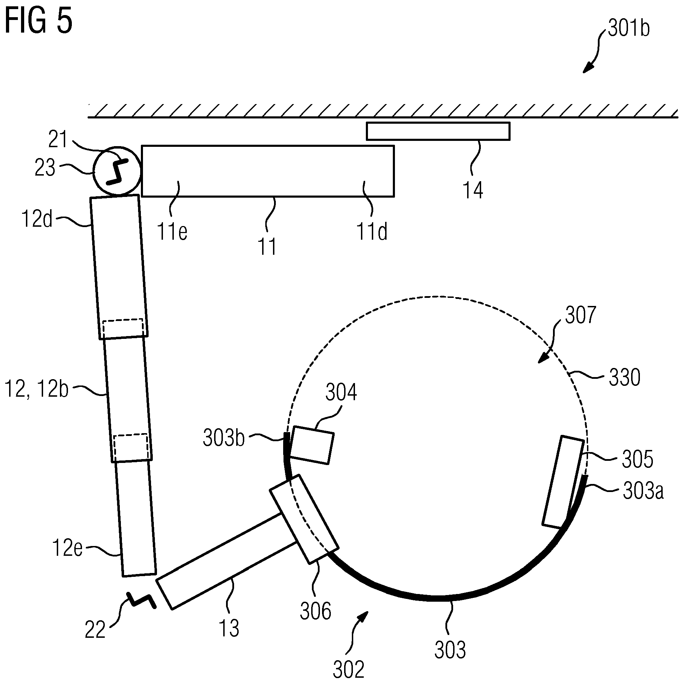

Fig. 4 zeigt eine erste Extremposition 301a des Röntgensystems 301, während Fig. 5 eine zweite Extremposition 301b des Röntgensystems 301 zeigt.

Das Röntgensystem 301 befindet sich somit in Fig. 4 in einer ersten Extremposition 301a und in Fig. 5 in einer zweiten Extremposition 301b. In den jeweiligen Extrempositionen 301a, 301b des Röntgensystems 301 befinden sich die Röntgeneinheit 307, also der Röntgenstrahler 304 und der Röntgendetektor 305, ebenfalls auf dem Drehkreis 330 in einer Extremposition.

Folgende Konfigurationen tragen dazu bei, dass die Röntgeneinheit 307 sich in Fig. 4 in der ersten Extremposition 301a befindet:
- Das erste Trägerelement 11 ist so konfiguriert, dass der Abstand zwischen der Aufhängevorrichtung 14 und dem ersten Verbindungselement 21, insbesondere der Abstand zwischen der Aufhängevorrichtung 14 und dem zweiten Ende 11e des ersten Trägerelements 11 minimal ist.
- Der Winkel des ersten Drehgelenks 23 zwischen dem ersten Trägerelement 11 und dem zweiten Trägerelement 12 ist größer im Vergleich zu einer Position auf dem Drehkreis 330, die nicht der ersten Extremposition 301a entspricht, wobei die Drehrichtung so definiert ist, dass der Winkel kleiner als 180° und positiv ist.
- Das zweite Trägerelement 12 ist so konfiguriert, dass der Abstand zwischen dem ersten Verbindungselement 21 und dem zweiten Verbindungselement 22, insbesondere der Abstand zwischen dem ersten Ende 12d des zweiten Trägerelements 12 und dem zweiten Ende 12e des zweiten Trägerelements 12 minimal ist.
- Die Haltevorrichtung 302 ist so verfahren, dass der Abstand vom ersten Ende 303a des C-Bogens 303 zum dritten Trägerelement 13, insbesondere der Abstand vom ersten Ende 303a des C-Bogens 303 zur Verbindungsvorrichtung 306 minimal ist. Die Teleskopeinheit 12b ist eingefahren.

Alternativ zum in Fig. 4 gezeigten Fall kann auch nur ein Teil der genannten Konfigurationen dazu beitragen, dass die Röntgeneinheit 307 sich in Fig. 4 in der ersten Extremposition 301a befindet.

Folgende Konfigurationen tragen dazu bei, dass die Röntgeneinheit 307 sich in Fig. 5 in der zweiten Extremposition 301b befindet:
- Das erste Trägerelement 11 ist so konfiguriert, dass der Abstand zwischen der Aufhängevorrichtung 14 und dem ersten Verbindungselement 21, insbesondere der Abstand zwischen der Aufhängevorrichtung 14 und dem zweiten Ende 11e des ersten Trägerelements 11 maximal ist.
- Der Winkel des ersten Drehgelenks 23 zwischen dem ersten Trägerelement 11 und dem zweiten Trägerelement 12 ist kleiner im Vergleich zu einer Position auf dem Drehkreis 330, die nicht der zweiten Extremposition 301b entspricht, wobei die Drehrichtung so definiert ist, dass der Winkel kleiner als 180° und positiv ist.
- Das zweite Trägerelement 12 ist so konfiguriert, dass der Abstand zwischen dem ersten Verbindungselement 21 und dem zweiten Verbindungselement 22, insbesondere der Abstand zwischen dem ersten Ende 12d des zweiten Trägerelements 12 und dem zweiten Ende 12e des zweiten Trägerelements 12 maximal ist.
- Die Haltevorrichtung 302 ist so verfahren, dass der Abstand vom ersten Ende 303a des C-Bogens 303 zum dritten Trägerelement 13, insbesondere der Abstand vom ersten Ende 303a des C-Bogens 303 zur Verbindungsvorrichtung 306 maximal ist. Die Teleskopeinheit 12b ist ausgefahren.

Alternativ zum in Fig. 5 gezeigten Fall kann auch nur ein Teil der genannten Konfigurationen dazu beitragen, dass die Röntgeneinheit 307 sich in Fig. 5 in der zweiten Extremposition 301b befindet.

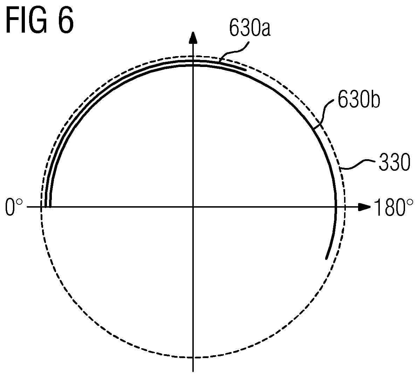

Fig. 6 zeigt einen Drehkreis der Haltevorrichtung 302. Der Drehkreis 330 entspricht dem orbitalen Drehkreis der Orbitalbewegung der Haltevorrichtung 302. Der Röntgenstrahler 304 und der Röntgendetektor 305, insbesondere auch die Röntgeneinheit 307 sind entlang der von der Form des C-Bogens 303 vorgegebenen ersten Bahn 630a ausschließlich mittels einer Bewegung des C-Bogens 303 relativ zur Verbindungsvorrichtung 306 verfahrbar.

Der Röntgenstrahler 304 und der Röntgendetektor 305 sind mittels des ersten Trägerelements 11, des Drehgelenks 23, des zweiten Trägerelements 12 und der Bewegung des C-Bogens 303 relativ zur Verbindungsvorrichtung 306 entlang einer zweiten Bahn 630b, die Teil des Drehkreises 330 ist und deren Bogenlänge einem zweiten Winkel entspricht, verfahrbar angeordnet. Der zweite Winkel der zweiten Bahn 630b ist größer als der erste Winkel der ersten Bahn 630a. Dies bedeutet insbesondere, dass die Haltevorrichtung 302 als solche, aber auch einzelne Teile der Haltevorrichtung 302 wie beispielsweise der C-Bogen 303, die Röntgeneinheit 307, der Röntgenstrahler 304 und/oder der Röntgendetektor 305 entlang der zweiten Bahn 630b verfahrbar ist. Der zweite Winkel beträgt mindestens 200°.

Obwohl die Erfindung im Detail durch das bevorzugte Ausführungsbeispiel näher illustriert und beschrieben wurde, ist die Erfindung nicht durch die offenbarten Beispiele eingeschränkt. Variationen hiervon können vom Fachmann abgeleitet werden, ohne den Schutzumfang der Erfindung, wie er durch die nachfolgenden Patentansprüche definiert wird, zu verlassen.

## Patentansprüche

1. Röntgensystem (301), aufweisend
- eine Tragevorrichtung (10, 210), welche
-- eine Aufhängevorrichtung (14, 214),
-- ein an der Aufhängevorrichtung angeordnetes erstes Trägerelement (11, 211),
-- ein zweites Trägerelement (12, 212),
-- ein erstes Verbindungselement (21, 221), wobei das zweite Trägerelement (12, 212) mittels des ersten Verbindungselements (21, 221) mit dem ersten Trägerelement (11, 211) verbunden ist,
-- ein drittes Trägerelement (13, 213) und
-- ein zweites Verbindungselement (22, 222) aufweist, wobei das dritte Trägerelement (13, 213) mittels des zweiten Verbindungselements (22, 222) mit dem zweiten Trägerelement (12, 212) verbunden ist, und
- eine mit dem dritten Trägerelement (13, 213) verbundene Haltevorrichtung (302), die einen C-Bogen (303), an dessen Enden ein Röntgenstrahler (304) und ein Röntgendetektor (305) angeordnet sind, und eine Verbindungsvorrichtung (306) aufweist,
- wobei das erste Verbindungselement (21, 221) und/oder das zweite Verbindungselement (22, 222) ein Drehgelenk (23, 223) aufweist, der Abstand zwischen der Aufhängevorrichtung (14, 214) und dem ersten Verbindungselement (21, 221) mittels des ersten Trägerelements (11, 211) veränderbar ist und der Abstand zwischen dem ersten Verbindungselement (21, 221) und dem zweiten Verbindungselement (22, 222) mittels des zweiten Trägerelements (12, 212) veränderbar ist,
- wobei der Röntgenstrahler (304) und der Röntgendetektor (305) entlang einer von der Form des C-Bogens (303) vorgegebenen ersten Bahn (630a), die Teil eines Drehkreises (330) ist und deren Bogenlänge einem ersten Winkel entspricht, mittels einer Bewegung des C-Bogens (303) relativ zur Verbindungsvorrichtung (306) verfahrbar angeordnet sind,
**dadurch gekennzeichnet,**
**dass** der Röntgenstrahler (304) und der Röntgendetektor (305) mittels des ersten Trägerelements (11, 211), des Drehgelenks (23, 223), des zweiten Trägerelements (12, 212) und der Bewegung des C-Bogens (303) relativ zur Verbindungsvorrichtung (306) entlang einer zweiten Bahn (630b), die Teil des Drehkreises (330) ist und deren Bogenlänge einem zweiten Winkel entspricht, verfahrbar angeordnet sind, wobei der zweite Winkel größer ist als der erste Winkel.

2. Röntgensystem (301) nach Anspruch 1, wobei das erste Verbindungselement (21, 221) das Drehgelenk (23, 223) aufweist.

3. Röntgensystem (301) nach Anspruch 1, wobei das zweite Verbindungselement (22, 222) das Drehgelenk (23, 223) aufweist.

4. Röntgensystem (301) nach einem der vorhergehenden Ansprüche, wobei das erste Trägerelement (11, 211) teleskopierbar und/oder relativ zur Aufhängevorrichtung (14, 214) verschiebbar ausgebildet ist.

5. Röntgensystem (301) nach einem der vorhergehenden Ansprüche, wobei das zweite Trägerelement (12, 212) teleskopierbar und/oder relativ zum ersten Verbindungselement (21, 221) verschiebbar ausgebildet ist.

6. Röntgensystem (301) nach einem der vorhergehenden Ansprüche, wobei das erste Trägerelement (11, 211) und das zweite Trägerelement (12, 212) teleskopierbar ausgebildet sind.

7. Röntgensystem (301) nach einem der vorhergehenden Ansprüche, wobei die Tragevorrichtung (10, 210) eine Systemachse (15, 315) aufweist, welche bei einer Aufhängung der Tragevorrichtung (10, 210) an einer Decke (309) oder einem Boden senkrecht auf der Decke (309) oder dem Boden steht und durch die Aufhängevorrichtung (14, 214) verläuft, wobei das erste Trägerelement (11, 211) an der Aufhängevorrichtung (14, 214) drehbar um die Systemachse (15, 315) gelagert ist.

8. Röntgensystem (301) nach einem der vorhergehenden Ansprüche, wobei das dritte Trägerelement (13, 213) relativ zum zweiten Verbindungselement (22, 222) um eine sich in Längsrichtung des dritten Trägerelements (13, 213) erstreckende Längsachse (13a) drehbar ausgebildet ist.

9. Röntgensystem (301) nach einem der vorhergehenden Ansprüche, wobei die Tragevorrichtung (10, 210) eine Systemachse (15, 315) aufweist, welche bei einer Aufhängung der Tragevorrichtung (10, 210) an einer Decke (309) oder einem Boden senkrecht auf der Decke (309) oder dem Boden steht und durch die Aufhängevorrichtung (14, 214) verläuft, wobei die Haltevorrichtung (302) entlang der Systemachse (15, 315) mittels der Tragevorrichtung (10, 210) höhenverstellbar ist.

10. Röntgensystem (301) nach Anspruch 9, wobei die Haltevorrichtung (302) entlang der Systemachse (15, 215) mittels des ersten Trägerelements (11, 211), des Drehgelenks (23, 223) und des zweiten Trägerelements (12, 212) höhenverstellbar ist.

11. Röntgensystem (301) nach einem der vorhergehenden Ansprüche, wobei der Abstand zwischen dem Röntgenstrahler (304) und dem Röntgendetektor (305) veränderbar ist.

12. Röntgensystem (301) nach einem der vorhergehenden Ansprüche, wobei der zweite Winkel mindestens 200° beträgt.

## Claims

1. X-ray system (301), having
- a carrying device (10, 210), which has
-- a suspension device (14, 214),
-- a first carrier element (11, 211) arranged on the suspension device,
-- a second carrier element (12, 212),
-- a first connecting element (21, 221), wherein the second carrier element (12, 212) is connected to the first carrier element (11, 211) by means of the first connecting element (21, 221),
-- a third carrier element (13, 213) and
-- a second connecting element (22, 222), wherein the third carrier element (13, 213) is connected to the second carrier element (12, 212) by means of the second connecting element (22, 222), and has
- a retaining device (302) which is connected to the third carrier element (13, 213) and has a C-arm (303), at the ends of which an X-ray tube assembly (304) and an X-ray detector (305) are arranged, and a connecting device (306),
- wherein the first connecting element (21, 221) and/or the second connecting element (22, 222) have/has a revolute joint (23, 223), the distance between the suspension device (14, 214) and the first connecting element (21, 221) is variable by means of the first carrier element (11, 211), and the distance between the first connecting element (21, 221) and the second connecting element (22, 222) is variable by means of the second carrier element (12, 212),
- wherein the X-ray tube assembly (304) and the X-ray detector (305) are arranged so as to be movable by means of a movement of the C-arm (303) relative to the connecting device (306) along a first path (630a) which is predefined by the shape of the C-arm (303) and is part of a circle of rotation (330) and the arc length of which corresponds to a first angle,
**characterised in that**
the X-ray tube assembly (304) and the X-ray detector (305) are arranged so as to be movable by means of the first carrier element (11, 211), the revolute joint (23, 223), the second carrier element (12, 212) and the movement of the C-arm (303) relative to the connecting device (306) along a second path (630b) which is part of the circle of rotation (330) and the arc length of which corresponds to a second angle, wherein the second angle is greater than the first angle.

2. X-ray system (301) according to claim 1, wherein the first connecting element (21, 221) has the revolute joint (23, 223).

3. X-ray system (301) according to claim 1, wherein the second connecting element (22, 222) has the revolute joint (23, 223).

4. X-ray system (301) according to one of the preceding claims, wherein the first carrier element (11, 211) is embodied as telescopic and/or as displaceable relative to the suspension device (14, 214).

5. X-ray system (301) according to one of the preceding claims, wherein the second carrier element (12, 212) is embodied as telescopic and/or as displaceable relative to the first connecting element (21, 221).

6. X-ray system (301) according to one of the preceding claims, wherein the first carrier element (11, 211) and the second carrier element (12, 212) are embodied as telescopic.

7. X-ray system (301) according to one of the preceding claims, wherein the carrying device (10, 210) has a system axis (15, 315) which, when the carrying device (10, 210) is suspended from a ceiling (309) or anchored to a floor, stands perpendicularly on the ceiling (309) or the floor and extends through the suspension device (14, 214), wherein the first carrier element (11, 211) is mounted on the suspension device (14, 214) so as to be rotatable about the system axis (15, 315) .

8. X-ray system (301) according to one of the preceding claims, wherein the third carrier element (13, 213) is embodied as rotatable relative to the second connecting element (22, 222) about a longitudinal axis (13a) extending in the longitudinal direction of the third carrier element (13, 213) .

9. X-ray system (301) according to one of the preceding claims, wherein the carrying device (10, 210) has a system axis (15, 315) which, when the carrying device (10, 210) is suspended from a ceiling (309) or anchored to a floor, stands perpendicularly on the ceiling (309) or the floor and extends through the suspension device (14, 214), wherein the retaining device (302) is height-adjustable along the system axis (15, 315) by means of the carrying device (10, 210).

10. X-ray system (301) according to claim 9, wherein the retaining device (302) is height-adjustable along the system axis (15, 215) by means of the first carrier element (11, 211), the revolute joint (23, 223) and the second carrier element (12, 212).

11. X-ray system (301) according to one of the preceding claims, wherein the distance between the X-ray tube assembly (304) and the X-ray detector (305) is variable.

12. X-ray system (301) according to one of the preceding claims, wherein the second angle equals at least 200°.

## Revendications

1. Système (301) à rayons X, comportant
- un dispositif (10, 210) porteur, qui a
-- un dispositif (14, 214) de suspension,
-- un premier élément (11, 211) de support monté sur le dispositif de suspension,
-- un deuxième élément (12, 212) de support,
-- un premier élément (21, 221) de liaison, le deuxième élément (12, 212) de support étant relié au premier élément (11, 211) de support au moyen du premier élément (20, 221) de liaison,
-- un troisième élément (13, 213) de support et
-- un deuxième élément (22, 222) de liaison, le troisième élément (13, 213) de support étant relié au deuxième élément (12, 212) de support au moyen du deuxième élément (22, 222) de liaison, et
- un dispositif (302) de maintien, qui est relié au troisième élément (13, 213) de support et qui a un arceau (303) en C, aux extrémités duquel sont montés un émetteur (304) de rayons X et un détecteur (305) de rayons X, et un dispositif (306) de liaison,
- dans lequel le premier élément (21, 221) de liaison et/ou le deuxième élément (22, 222) de liaison a une rotule (23, 223), la distance entre le dispositif (14, 214) de suspension et le premier élément (21, 221) de liaison peut être modifiée au moyen du premier élément (11, 211) de support et la distance entre le premier élément (21, 221) de liaison et le deuxième élément (22, 222) de liaison peut être modifiée au moyen du deuxième élément (12, 212) de support,
- dans lequel l'émetteur (304) de rayons X et le détecteur (305) de rayons X sont montés de manière à pouvoir se déplacer par rapport au dispositif (306) de liaison, au moyen d'un déplacement de l'arceau (303) en C, le long d'une première trajectoire (630a), qui est donnée par la forme de l'arceau (303) en C, qui fait partie d'un cercle (330) de giration et dont la longueur d'arc correspond à un premier angle,
**caractérisé**
**en ce que** l'émetteur (304) de rayons X et le détecteur (305) de rayons X sont disposés de manière à pouvoir se déplacer au moyen du premier élément (11, 211) de support, de la rotule (23, 223), du deuxième élément (12, 212) de support et du déplacement de l'arceau (303) en C par rapport au dispositif (306) de liaison, le long d'une deuxième trajectoire (630b), qui fait partie du cercle (330) de giration et dont la longueur d'arc correspond à un deuxième angle, le deuxième angle étant plus grand que le premier angle.

2. Système (301) à rayons X suivant la revendication 1, dans lequel le premier élément (21, 221) de liaison a la rotule (23, 223).

3. Système (301) à rayons X suivant la revendication 1, dans lequel le deuxième élément (22, 222) de liaison a la rotule (23, 223).

4. Système (301) à rayons X suivant l'une des revendications précédentes, dans lequel le premier élément (11, 211) de support est constitué de manière télescopique et/ou de manière à pouvoir être déplacé par rapport au dispositif (14, 214) de suspension.

5. Système (301) à rayons X suivant l'une des revendications précédentes, dans lequel le deuxième élément (12, 212) de support est télescopique et/ou est constitué de manière à pouvoir être déplacé par rapport au premier élément (21, 221) de liaison.

6. Système (301) à rayons X suivant l'une des revendications précédentes, dans lequel le premier élément (11, 211) de support et le deuxième élément (12, 212) de support sont constitués de manière télescopique.

7. Système (301) à rayons X suivant l'une des revendications précédentes, dans lequel le dispositif (10, 210) porteur a un axe (15, 315) de système, qui, lors d'une suspension du dispositif (10, 210) porteur s'élève sur un plafond (309) ou un plancher perpendiculairement au plafond (309) ou au plancher et s'étend dans le dispositif (14, 214) de suspension, le premier élément (11, 211) de support étant monté tournant autour de l'axe (15, 315) du système sur le dispositif (14, 214) de suspension.

8. Système (301) à rayons X suivant l'une des revendications précédentes, dans lequel le troisième élément (13, 213) de support est constitué tournant par rapport au deuxième élément (22, 222) de liaison autour d'un axe (13a) longitudinal s'étendant dans la direction longitudinale du troisième élément (13, 213) de support.

9. Système (301) à rayons X suivant l'une des revendications précédentes, dans lequel le dispositif (10, 210) porteur a un axe (15, 315) de système, qui, lors d'une suspension du dispositif (10, 210) porteur, s'élève sur un plafond (309) ou un plancher perpendiculairement au plafond (309) ou au plancher et s'étend dans le dispositif (14, 214) de suspension, le dispositif (302) de maintien étant réglable en hauteur le long de l'axe (15, 315) du système, au moyen du dispositif (10, 210) porteur.

10. Système (301) à rayons X suivant la revendication 9, dans lequel le dispositif (302) de maintien est réglable en hauteur le long de l'axe (15, 215) du système, au moyen du premier élément (11, 211) de support, de la rotule (23, 223) et du deuxième élément (12, 212) de support.

11. Système (301) à rayons X suivant l'une des revendications précédentes, dans lequel la distance entre l'émetteur (304) de rayons X et le détecteur (305) de rayons X est réglable.

12. Système (301) à rayons X suivant l'une des revendications précédentes, dans lequel le deuxième angle a une valeur d'au moins 200°.
